# EUROPEAN PATENT APPLICATION

(11) **EP 4 541 288 A1**
(43) Date of publication of application: **23.04.2025**
(21) Application number: 23306813.9
(22) Date of filing: 17.10.2023
(51) Int. Cl.: A61B 8/06

(54) **CARDIAC ULTRASOUND VISUALIZATION METHOD**

(71) Applicant: Koninklijke Philips N.V., 5656 AG Eindhoven (NL)
(72) Inventor: BOUYRIE, Mathieu, 5656 EINDHOVEN (NL); ROUET, Jean-Michel, 5656 EINDHOVEN (NL); BONNEFOUS, Odile, 5656 EINDHOVEN (NL)
(74) Representative: Philips Intellectual Property & Standards

(57) **Abstract**

A method for visualization of a heart valve and blood flow therethrough for the purpose of assessing valve regurgitation. The visualization is generated based on both 3D B-mode data and 3D Doppler data. The visualization comprises generating a combined rendering of each of a heart valve structure, an upstream converging flow region and an output jet flow region. Rendering parameters applied in rendering each of these structures may preferably be independently adjustable to allow for tuning the visualization. The rendering may be performed by a renderer operable to render both raster data and mesh data.

## Description

### FIELD OF THE INVENTION

This invention relates to a method for visualization of cardiac ultrasound data, for example cardiac ultrasound data representative of a heart valve region.

### BACKGROUND OF THE INVENTION

Cardiac ultrasound imaging is applied for a range of different diagnostic purposes.

One example application is for analysis of regurgitant flow through a heart valve (e.g. aortic valve, mitral valve, tricuspid valve, pulmonary valve). For imaging the flow itself, the most common mode of ultrasound imaging is Color Flow Doppler imaging. This is favorable due to its relative low cost and small space footprint (e.g. when compared to alternatives such as MRI).

For imaging the valve, 3D ultrasound imaging can be used to produce 3D B-mode images. 3D Doppler color images can be obtained of the blood flow to thereby create an overall image depicting the heart structure filled with flow information within the cavities and through the valves between them. Dedicated flow models exist in the state of the art to enable quantification of the leaks through the mitral valve in case of regurgitation during the systolic phase.

However, state of the art ultrasound visualization techniques in the context of regurgitant flow analysis have weaknesses.

One challenge is accurately and fully obtaining the 3D topology of the heart valve from the acquired 3D B-mode data. This may be due to the size of the image being insufficient relative to the image resolution, or due to the orientation of the valve being perpendicular to the ultrasound beam, resulting in poor echo return.

Even when there is no loss of valve signal in the B-mode data, tuning visualization parameters for rendering a 2D image from the 3D data is not a simple task. If parameters are too strong, the image may show presence of phantom holes which do not exist in the true anatomy, while if parameters are too weak, holes which are present may be missed.

An ideal visualization of the 3D ultrasound data would include a representation of the topology and physics of the valve, the blood flow, and preferably the ventricle structure, and atrium structure. Such a visualization enables a clinician to assess the valve accurately, taking into account the full anatomical context. More particularly, an ideal visualization would enable representation of a plurality of the following:
the topology and the location of the valve;
the morphology, the size, and the location of each orifice in the valve;
the interaction between the blood flow and the valve;
the convergence of the blood flow at the entrance of the orifice in the valve;
incoming blood flow through the orifice of the valve;
the output flow jet in the atrium due to the regurgitation.

State of the art visualization tools may enable visualization of a selected one of these aspects, but not a plurality of them, and not all of them together.

### SUMMARY OF THE INVENTION

The invention is defined by the claims.

In accordance with an aspect of the invention, there is provided a method for cardiac valve visualization using ultrasound data. Another aspect provides a processing device configured to perform the method. Another aspect provides a computer program product comprising computer program code configured, when run on a processor, to cause the processor to perform the method.

The method comprises receiving 3D B-mode ultrasound data of a cardiac region containing a heart valve structure.

The method further comprises receiving 3D Doppler ultrasound data of the cardiac region.

The method further comprises generating a 2D image based on the B-mode data and Doppler data.

The generating the 2D image comprises applying image rendering.

The image rendering may be for deriving a combined rendering of: a heart valve structure in the cardiac region, and an output jet flow emitted from the heart valve, and/or an upstream converging flow region upstream of the valve. This may for example be a region which contains at least a isovelocity surface of blood flow.

In a preferred embodiment, the combined rendering may further comprise a visualization of at least a portion of the structure of one or more cardiac cavities (preferably an atrium and/or a ventricle).

The method may comprise deriving a 3D raster of the heart valve structure from the 3D B-mode image data.

The method may comprise deriving a 3D mesh of the output jet flow using the 3D Doppler ultrasound data and/or deriving a 3D raster of the output jet flow using the 3D Doppler ultrasound data. The method may comprise deriving a 3D mesh of the upstream converging flow region using the 3D Doppler ultrasound data and/or deriving a 3D raster of the upstream converging flow region using the 3D Doppler ultrasound data.

The image rendering may comprise applying image rendering to: the 3D raster of the heart valve structure, a 3D mesh or 3D raster of the output jet flow and a 3D mesh or 3D raster of the upstream (converging) flow region to generate the 2D image.

The generating the 2D image may comprise identifying in the B-mode data and/or Doppler data a heart valve structure. The generating the 2D image may comprise identifying in the B-mode data and/or Doppler data an output jet flow region. The generating the 2D image may comprise identifying in the B-mode data and/or Doppler data an upstream converging flow region.

For example, the generating the 2D image may comprise identifying a 3D region of the B-mode data occupied by the heart valve structure to thereby yield a 3D raster representation of the heart valve. The generating the 2D image may comprise identifying an output jet flow region within the cardiac region containing a fluid jet output from the heart valve. This may be based on processing of at least the Doppler ultrasound data. It may additionally be based on processing of the 3D B-mode data. The generating the 2D image may comprise identifying an upstream converging flow region within the cardiac region, wherein an upstream converging flow region is a region containing at least an isovelocity surface of blood flow. This may be based on processing of at least the Doppler ultrasound data. It may be based additionally on processing of the 3D B-mode data.

The method may comprise performing a rendering process to render a 2D image which includes a visualization of each of the heart valve structure, the output jet flow region, and the upstream converging flow region.

The rendering process may include applying rendering to the 3D raster representation of the heart valve for realizing in the 2D image a 2D visualization of the heart valve structure.

It is proposed in accordance with embodiments of the present invention to render a 2D visualization of the valve and surrounding region based using a combination of 3D B-mode data and 3D Doppler data.

Furthermore, in at least one set of embodiments, the valve is rendered by applying volume rendering to a 3D raster of the B-mode echo data (rather than to a mesh segmented from the echo data). This means that the rendering of the heart valve region takes account of internal structure information and also advantageously allows for an extra degree of freedom in configuring the visual properties of the rendering, by adjusting the voxel transparency transfer function in the volume rendering of the 3D raster. This is not an option when rendering a mesh. This advantageously allows for optimizing visibility of the most relevant structural elements of the heart valve structure and for optimizing relative visibility of the valve and the flow streams. For example, this allows for achieving better alignment between the jet and the orifice.

A 3D raster means a (3D) voxel dataset. A 3D raster (or voxel dataset) is distinguished from other types of 3D image representation such as a 3D vector image, or a 3D mesh or other types of 3D segmentation.

The method may comprise independently adjusting one or more rendering parameters applied in rendering each of the heart valve structure, the output jet flow region, and the upstream converging flow region.

This allows for improved visualization by enabling independently tunable rendering for each of the valve, the regurgitant jet and the convergence flow region, via independently tunable visualization parameters. This allows for optimizing visibility of the different elements of the valve and the flow.

In the state of the art, there is no known easy way to visualize 3D echo and color Doppler data so as to provide a clear representation of any mitral regurgitation that may be present. This is true for both TTE and TEE imaging. However, distinguishing and identifying these components in the process of regurgitation is fundamental for the making of a diagnosis.

In some embodiments, the one or more rendering parameters may include at least a relative transparency level of each of the heart valve structure, the output jet flow region, and the upstream converging flow region in the final generated image.

In some embodiments, the one or more rendering parameters may include one or more of: transparency, smoothness, color, and/or fading applied to each of the heart valve structure, the output jet flow region, and the upstream converging flow region.

The rendering of one or more of the heart valve structure, the output jet flow region, and the upstream converging flow region may include applying a rendering transfer function to ultrasound data. In some embodiments, the one or more rendering parameters may include one or more parameters of the rendering transfer function. For example, it may include a parameter of a rendering transfer function used in deriving a rendering of the 3D raster of the heart valve structure.

In some embodiments, the adjusting the one or more rendering parameters may comprise receiving user input from a user interface indicative of settings for at least one of the one or more rendering parameters and adjusting the at least one rendering parameter based on the user input. Alternatively, the adjustment may be performed automatically.

In some embodiments, the rendering the heart valve structure may comprises applying a transfer function to voxels of the raster representation of the heart valve, wherein the transfer function defines one or more visualization properties, such as a transparency level, for each voxel of the raster as a function of voxel value. The one or more rendering parameters include one or more parameters of the transfer function. For example, the one or more rendering parameters may include a threshold level or value for the transfer function. For example, the threshold level or value may define a voxel value above which voxels of the raster representation are included in the rendering and below which voxels of the raster representation are not included in the rendering, i.e. below which the transparency of a voxel is 100% and above which the transparency of a voxel is less than 100%.

In some embodiments, the identifying of the output jet flow region may comprise segmenting the output jet flow region in the Doppler ultrasound data. The rendering of the output jet flow region may comprise generating a 3D mesh of an outline of the output jet flow region based on the segmentation, and applying volume rendering to the mesh.

Instead of generating a 3D mesh, in some embodiments, the rendering of the output jet flow region comprises applying volume rendering to a 3D raster representation of the output jet flow region within the Doppler data.

In some embodiments, the identifying the upstream converging flow region comprises segmenting the upstream converging flow region in the Doppler data. The rendering of the upstream converging flow region may comprise: generating a 3D mesh of the upstream converging flow region based on the segmentation, and applying volume rendering to the mesh.

Instead of generating a 3D mesh, in some embodiments, the rendering the upstream converging flow region comprises applying volume rendering to a 3D raster representation of the upstream converging flow region within the Doppler data.

In some embodiments, identifying the 3D region of the B-mode data occupied by the heart valve structure comprises segmenting the heart valve structure in the B-mode data.

In some embodiments, identifying the 3D region of the B-mode data occupied by the heart valve structure comprises computing a 3D mask based on the segmentation, the 3D mask distinguishing the 3D region occupied by the heart valve structure from a background region.

In some embodiments, identifying of the output jet flow region and the upstream converging flow region comprises applying a flow dynamics model to the Doppler ultrasound data. The flow dynamics model may be adapted to determine an isovelocity surface of blood flow upstream of the heart valve. The flow dynamics model may be adapted to determine a blood flow path through the valve. The flow dynamics model may be adapted to determine an output jet from the valve.

The velocity surface may be a proximal isovelocity surface area (PISA).

An example flow dynamics model suitable for this purpose is described in detail in the document WO 2023/020920 A1.

A further aspect of the invention provides a processing device, comprising one or more processors configured to: receive: 3D B-mode ultrasound data of a cardiac region containing a heart valve structure, and receive 3D Doppler ultrasound data of the cardiac region; identify a 3D region of the B-mode data occupied by the heart valve structure to yield a 3D raster representation of the heart valve; identify an output jet flow region within the cardiac region containing a fluid jet output from the heart valve, based on processing of at least the Doppler ultrasound data; identify an upstream converging flow region within the cardiac region, wherein an upstream converging flow region is a region containing at least an isovelocity surface of blood flow, based on processing of at least the Doppler ultrasound data; and perform a rendering process to render a 2D image which includes a visualization of each of the heart valve structure, the output jet flow region, and the upstream converging flow region.

The rendering process may include applying rendering to the 3D raster representation of the heart valve to yield in the 2D image a 2D visualization of the heart valve structure.

The processing device may in some embodiments be further configured for independently adjusting one or more rendering parameters applied in rendering each of the heart valve structure, the output jet flow region, and the upstream converging flow region. The one or more parameters may include a transparency level applied to each of the heart valve structure, the output jet flow region, and the upstream converging flow region.

The processing device may comprise a receive module for receiving the 3D B-mode ultrasound data and the 3D Doppler ultrasound data. The processing device may comprise a 3D render module for performing the identifying steps and the rendering process. The processing device may include a render tuning module for independently adjusting the one or more rendering parameters.

A further aspect of the invention is a system, comprising: a processing device in accordance with any example or embodiment in this disclosure, or in accordance with any claim of this application; and an ultrasound acquisition apparatus, operable to acquire 3D B-mode data and 3D Doppler data.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 is a block diagram of an example processing device and system in accordance with one or more embodiments of the invention;
Figs. 3 and 4 illustrate example renderings of a mitral valve, upstream converging flow region and an outflow jet flow region;
Figs. 5 and 6 illustrate further example renderings of a mitral valve, upstream converging flow region and an outflow jet flow region;
Fig. 7 illustrates a processing flow of an example method in accordance with a set of embodiments of the invention;
Fig. 8 illustrates adjustment of a transparency parameter of a rendering of a valve structure;
Fig. 9 illustrates adjustment of a smoothness parameter of a rendering of a valve structure;
Fig. 10 illustrates adjustment of a fading parameter of a rendering of a valve structure;
Fig. 11 illustrates adjustment of a thresholding parameter of a rendering of a valve structure; and
Fig. 12 illustrates example renderings across a sectional plane through a valve structure.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for visualization of a heart valve and blood flow therethrough for the purpose of assessing valve regurgitation. The visualization is generated based on both 3D B-mode data and 3D Doppler data. The visualization comprises generating a combined rendering of each of a heart valve structure, an upstream converging flow region and an output jet flow region. Rendering parameters applied in rendering each of these structures may preferably be independently adjustable to allow for tuning the visualization. The rendering may be performed by a renderer operable to render both raster data and mesh data. The rendering of the valve structure may in some embodiments comprise applying rendering to a raster representation of the valve structure, e.g. derived from 3D B-mode data. This enables internal structure information to be taken into account in generating the visualization.

At least one set of embodiments of the present invention may provide a tunable tool for enabling diagnostic analysis by a user of a mitral regurgitation. This is achieved through the visualization of the main elements involved in the phenomenon, which may include one or more of:
The 3D volume of the mitral valve, e.g. derived from 3D echo data (e.g. B-mode data).
Parts of the 3D volumes of the left atrium, and/or the left ventricle, e.g. derived from 3D echo data (e.g. B-mode data).

The upstream convergence flow region. This is a region of blood flow upstream of the valve where flow paths converge. It is typically characterized by containing a flow isovelocity surface. Visualization of this region may be derived at least in part from 3D color Doppler data. Visualization may be derived using a flow model or other regurgitation analysis which may be configured to compute an isovelocity geometry of the incoming flow, and/or 3D flow streamlines. For example, using a flow model, velocity vectors may be produced to represent the flow through the valve.

Output jet flow region. Visualization of this may be derived from at least the 3D color Doppler data. Visualization may be derived using a flow model or other regurgitation analysis method which may be configured to derive a 3D volume geometry representing a segmentation of the jet and/or 3D streamlines of the jet.

Visualizing two or more of these components all together allows a practitioner to better understand where the valve orifice is located and its status e.g. relative to a Carpentier classification.

Any of the elements enumerated above may be represented using a 3D renderer and visualization of each may be independently tunable according to one or more of transparency, data threshold, smoothness, color, fading factors. The tuning may be configurable via a user interface. The user interface may permit tuning simultaneously with display of a rendered image. Alternatively, tuning could be performed automatically.

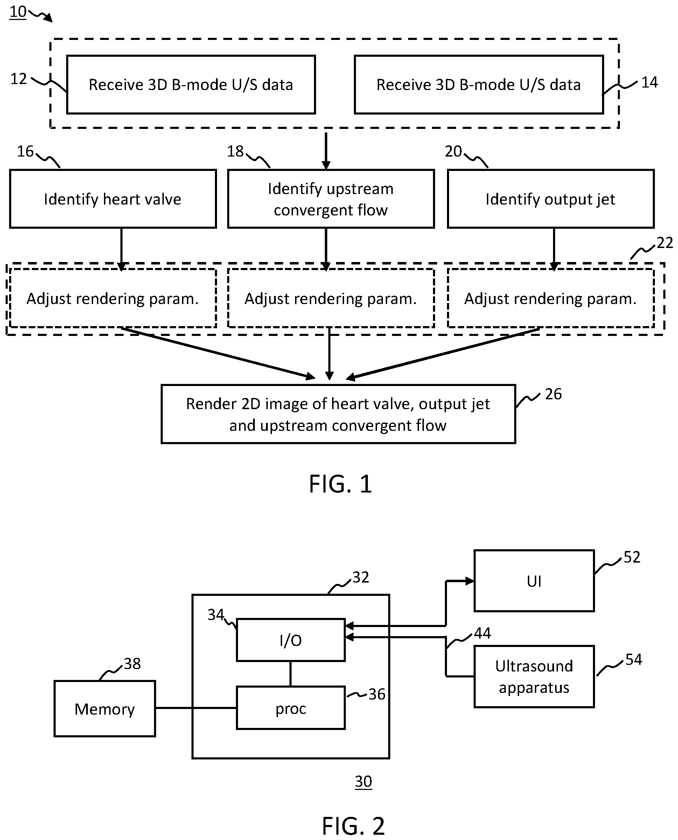

Fig. 1 outlines in block diagram form steps of an example method according to one or more embodiments. The steps will be recited in summary, before being explained further in the form of example embodiments.

Provided is a method 10 for cardiac valve visualization using ultrasound data.

The method comprises receiving 12 three-dimensional B-mode ultrasound data of a cardiac region containing a heart valve structure. The method further comprises receiving 14 three-dimensional Doppler ultrasound data of the cardiac region.

The method 10 further comprises generating an image based on the B-mode data and Doppler data. The generating the image comprises applying image rendering. The image rendering may be for rendering a 2D image. The image rendering may be for deriving a combined rendering of: a heart valve structure in the cardiac region, and an output jet flow region emitted from the heart valve, and/or an upstream converging flow region upstream of the valve, for example containing at least a isovelocity surface of blood flow. In a preferred embodiment, the combined rendering may further comprise at least a portion of the structure of one or more cardiac cavities (preferably a left atrium and/or left ventricle).

With reference to the example method of Fig. 1, the method may comprise identifying 16 a 3D region of the B-mode data occupied by the heart valve structure. This may be used to obtain a 3D raster representation of the heart valve. The method may comprise identifying 20 an output jet flow region within the cardiac region containing a fluid jet output from the heart valve, based on processing of at least the Doppler ultrasound data. The method may comprise identifying 18 an upstream converging flow region within the cardiac region, for example wherein an upstream converging flow region is a region containing at least an isovelocity surface of blood flow, for example based on processing of at least the Doppler ultrasound data.

With reference to Fig. 1, the method comprises performing a rendering process 26 to render an image, e.g. a 2D image, which includes a visualization of each of the heart valve structure, the output jet flow region and the upstream converging flow region.

The rendering process may include applying rendering to the 3D raster representation of the heart valve for realizing in the 2D image a 2D visualization of the heart valve structure.

Optionally, and as illustrated in Fig. 1, the method 10 may comprise independently adjusting 22 one or more rendering parameters applied in rendering each of the heart valve structure, the output jet flow region, and the upstream converging flow region. This allows for finetuning of the visualization of each of the heart valve structure, the upstream converging flow region and the output jet flow region in the final image. For example, in some embodiments, the one or more rendering parameters may include at least a transparency level of each of the heart valve structure, the output jet flow region and the upstream converging flow region in the generated 2D image. For example, this may be a relative transparency of the heart valve, the upstream converging flow region and the output jet flow region in the final image. In some embodiments, the one or more rendering parameters include one or more of: transparency, smoothness, color, and/or fading applied to each of the heart valve structure, the output jet flow region, and the upstream converging flow region.

The rendering of one or more of the heart valve structure, the output jet flow region and the upstream converging flow region may include applying a rendering transfer function to ultrasound data. In some embodiments, the one or more configurable rendering parameters may include one or more parameters of the rendering transfer function.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 2, the system 30 further comprises a user interface 52. In some embodiments, the adjusting the one or more rendering parameters may comprise receiving user input from the user interface indicative of settings for at least one of the one or more rendering parameters and adjusting the at least one rendering parameter based on the user input. Alternatively, the adjustment may be performed automatically.

In the illustrated example of Fig. 2, the system 30 further comprises an ultrasound acquisition or imaging apparatus 54 for acquiring 3D ultrasound data 44. The ultrasound data 44 includes 3D B-mode data and 3D Doppler data.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

With regards to generating the combined rendering, the process may be understood in at least some embodiments to comprise the following basic steps.

A set of ultrasound data is received for a cardiac region of a patient. This may be received from an ultrasound acquisition apparatus 54, e.g. in real time, or it may be received from a datastore storing previously acquired ultrasound data. The ultrasound data includes both 3D Doppler data and 3D B-mode data. An aim is to render a representation of the volume which has been scanned in a way that permits individual configuration of rendering settings for each of the heart valve structure, and a flow through the heart valve, e.g. including the upstream convergence flow region and the output jet flow region. To this end, a first process may involve identifying and/or isolating and/or extracting relevant volumetric portions of the received data which correspond to the different elements to be visualized. This can be achieved through application of one or more suitable segmentation algorithms to the B-mode data and/or to the Doppler data.

With regards to identifying relevant regions corresponding to the blood flow, it may include applying a flow analysis module or algorithm, for instance a regurgitation analysis module or algorithm, capable of analyzing the Doppler data to identify flow geometry and/or flow paths, and optionally to compute one or more flow-related quantities. Such software modules are known in the art, e.g. Philips QLAB, which offers advanced Doppler and flow quantification tools. The output of such analysis modules includes for example a representation of the geometry and/or flow paths or contours of the blood flow. They may additionally provide an output representation of a geometry or shape of orifice(s) of the valve (since an outer envelope shape of the flow as it passes through the valve will reflect the shape of the orifice).

An example flow analysis model suitable for performing these functions is outlined for instance in the document WO 2023/020920 A1. By way of brief summary, the flow analysis method of WO 2023/020920 A1 comprises receiving as input both B-mode ultrasound data 62 and Doppler ultrasound data 64. The method comprises generating a model of the mitral valve based on the B-mode data. The method further comprises generating a model of the orifice based on the model of the mitral valve and the Doppler data. The method further comprises generating a blood flow model of the mitral regurgitation based on the model of the orifice. The method may further comprise comparing the blood flow model to the Doppler data and changing the model of the orifice such that the blood flow model is changed to match the Doppler data. For example, the model of the orifice may be changed to have at least one of a different shape, a different position, or a different orientation. The reader is referred to WO 2023/020920 A1 for a detailed description of implementation details for this example method.

In addition to the heart valve structure, the upstream converging flow region and the output jet flow region, the method may include identifying and/or isolating and/or extracting volumetric portions of the received ultrasound data which correspond to at least portions of one or more heart chambers such as a left ventricle, and/or a left atrium.

Once each of the relevant elements to be visualized have been identified and/or isolated and/or extracted, an image containing the elements needs to be generated, and this involves applying image rendering.

As will be understood by the skilled person, there are a wide variety of different possible techniques for rendering the extracted elements of the ultrasound data. At least two will be briefly discussed. One approach is raster-based rendering. In raster based rendering, a rendering algorithm is applied to a 3D raster representation of the relevant element to be visualized, i.e. one or more of the heart valve structure, the upstream converging flow region and the output jet flow region. A 3D raster representation can be understood as a 3D voxel representation of the element. A 3D raster representation of the heart valve region can for example be obtained by simply extracting the volumetric region of the 3D B-mode data which contains the heart valve structure. A 3D raster representation of the different elements of the flow can also be extracted from either the 3D Doppler data, the B-mode data or a flow analysis module as mentioned above. Rendering of a 3D raster can be performed using a variety of techniques such as for example direct volume rendering, volume slicing, indirect volume rendering, maximum intensity projection (MIP), multiplanar reconstruction (MPR), shading models, and so on. Preferably, the rendering is a volume rendering method so as to provide a 2D depiction of the 3D geometry of the different elements (i.e. excluding MPR rendering). With regards to direct volume rendering, as will be well known to the skilled reader, this comprises performing ray casting through the voxels of the 3D raster and applying transfer function(s) to voxels encountered by each ray projected through the raster to arrive at a set of pixel values of a 2D image frame through which each ray passes before passing through the raster. The transfer function(s) map voxel values to colors and opacities. This way, certain features within the volume can be highlighted or made transparent.

Instead of raster-based rendering, an alternative is mesh-based rendering. In mesh-based rendering, a mesh is constructed which represents the 3D outer shape/morphology of a structure or volumetric region. Thus, this typically only represents the outer geometry of an element. The mesh is based on polygonal representations, often comprised of vertices, edges, and faces. These polygonal models are defined by a collection of vertices that form the geometric shape of the object and can be triangles, quadrilaterals, or other polygonal shapes. The constructed mesh can then be rendered using a variety of techniques such as orthographic or perspective projection, ray tracing, ray casting, scanline rendering, or rasterization. Thus, mesh based rendering may involve two processes (after the identification or extraction of the relevant element from the data, e.g. segmentation): construction of a mesh which represents the outer geometry of the relevant element (e.g. the heart valve structure, the upstream converging flow region, and/or the output jet flow region), followed by rendering applied to the mesh. Constructing the mesh from a segmented volumetric region can employ a range of techniques such as marching cubes, or surface nets or dual contouring.

Thus any of the heart valve structure, the upstream converging flow region, and the output jet flow region may be rendered using raster-based rendering or using mesh-based rendering.

In either case, the rendering process involves various configurable rendering parameters, adjustment of which will affect how the relevant element being rendered will appear in the final 2D image.

In at least some embodiments, the rendering process 26 comprises independently configuring or adjusting rendering parameters applied in rendering each of the heart valve structure, the upstream converging flow region, and the output jet flow region.

Examples of adjustable rendering parameters common to both raster based rendering and mesh-based rendering include the following:
opacity/transparency (how see-through or solid the rendered element appears in the image);
   brightness;
contrast;
color / color mapping (this associates defined colors with defined ranged of values or ranges of data);
lighting (includes parameters related to virtual light sources, such as their position, intensity, and color);
clipping planes (this allows for the selection of specific sections or slices of the data to be visualized, while omitting others);
smoothness / shading model (determines how surfaces respond to light, affecting the perception of smoothness or roughness);
sampling rate (especially relevant for volume (raster) rendering, this parameter dictates how often the data is sampled along the viewing rays);
texture mapping (involves applying a 2D image (texture) onto a rendered surface, affecting its appearance);
fading (modifies the level of fading of objects in the scene)
depth cueing (modifies the appearance of objects based on their depth in the scene, e.g. fading with respect to distance, to enhance depth perception).
blending mode (determines how overlapping objects combine visually, such as additive or average blending);
resolution (the detail level of the rendering).

The method 10 may comprise independently adjusting any one or more of the above-mentioned rendering parameters for each of the heart valve structure, the upstream converging flow region, and the output jet flow region.

By visualizing the flow path through the valve complex as well as visualizing the regurgitant valve structure, diagnostic analysis of the valve lesion is enhanced. Furthermore, combining a rendering using the B-mode data (of the valve structure) and another rendering using a flow model (discussed above) provides complementary representation of the lesion and its functional effects. There is thus proposed a method for displaying the flow pattern in conjunction with the anatomic valve, such that the interaction between the flow and the valve structure may be clearly understood.

In a preferred set of embodiments, rendering of the valve structure is performed with raster-based rendering. This is advantageous because it allows internal structure information to be taken into account in the visualization.

In some embodiments, identifying/extracting of the heart valve region may be performed using a combination of segmentation and voxel masking. For example, the identifying the 3D region of the B-mode data occupied by the heart valve structure may comprise: segmenting the heart valve structure in the B-mode data, and computing a 3D mask based on the segmentation, the 3D mask distinguishing the 3D region occupied by the heart valve structure from a background region.

To explain further, to separate the valve from the remainder of the echo data volume, a 3D mask may be introduced for selecting the valve signal within the broader heart valve region. This way, the region inside the 3D mask region may be rendered differently than the region outside of the 3D mask, meaning that visibility of the valve region can be enhanced, e.g. highlighted, relative to the surrounding region. So that it gets better visibility and better highlighting.

To compute the 3D mask, a segmentation algorithm may be first employed to yield a segmentation of the valve structure. From the segmentation, the following procedure may be applied. A set of points is extracted uniformly distributed on the valve. For example, in the case of triangular mesh, all the vertices are taken. Alternatively, segmentation of a voxel region can be performed and points sampled from the voxels or the entire voxel set used. Next, an identification of the main orientation or axes of the valve can be performed, for example by applying a principal component analysis (PCA) is to identify the horizontal axis (e.g. the shortest axis). To the collection of points, two new sets of points are added. These new points are created by translating the original points along the shortest axis identified by PCA. This yields an expanded collection of points with additional points on both sides of the original set, along the short axis. Next, all points can be dilated along the two other axes. After dilation, the set of points is processed by a convex envelope (or convex hull) algorithm which yields identification of the smallest convex shape that encompasses all the points. The mask is then formed by selecting of all voxels inside the convex envelope. This thus yields a 3D mask that fits around the valve structure.

The above-described rendering of each of the heart valve structure (rendered from the B-mode data), the upstream converging flow region (rendered using at least the Doppler data) and the output jet flow region (rendered using at least the Doppler data) may be incorporated into a single composite rendering operation, to yield a composite rendered 2D image, as follows:

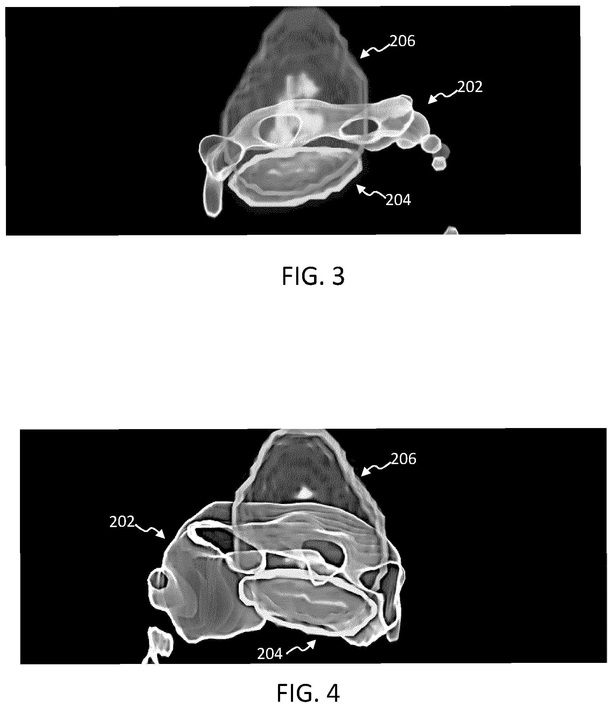

Fig. 3 and Fig. 4 shows example renderings of mitral regurgitation which includes a representation of each of the heart valve structure 202, the upstream converging flow region 204 toward the orifice, and the output jet flow region 206. Fig. 3 and Fig. 4 show the same regurgitation from opposite sides. The heart valve structure in this example was rendered from a raster representation of the heart valve structure.

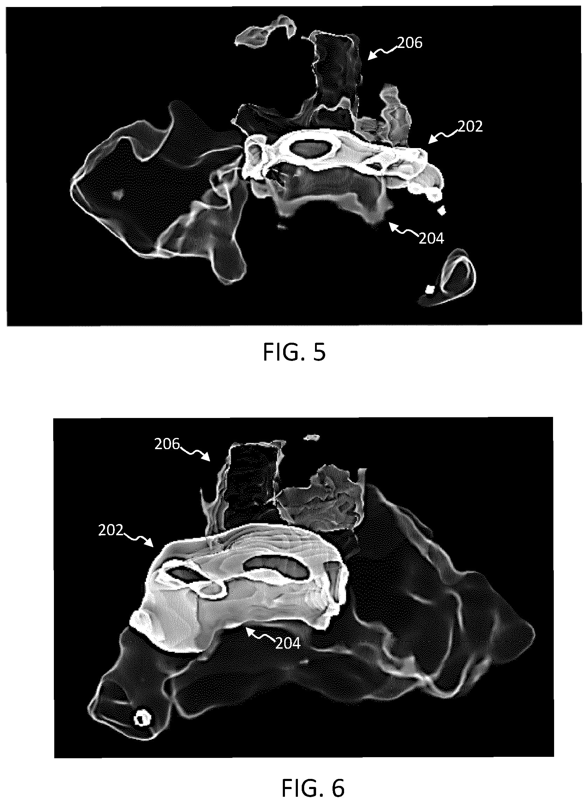

Fig. 5 and Fig. 6 show a further example rendering of mitral regurgitation which includes a representation of each of the heart valve structure 202, the upstream converging flow region 204 toward the orifice, and the output jet flow region 206. Fig. 5 and Fig. 6 show the same regurgitation from opposite sides. This visualization also shows portions of the left atrium and left ventricle. The heart valve structure, and the portions of the left atrium and left ventricle, were rendered from a raster representation of the heart valve structure, and the portions of the left atrium and left ventricle.

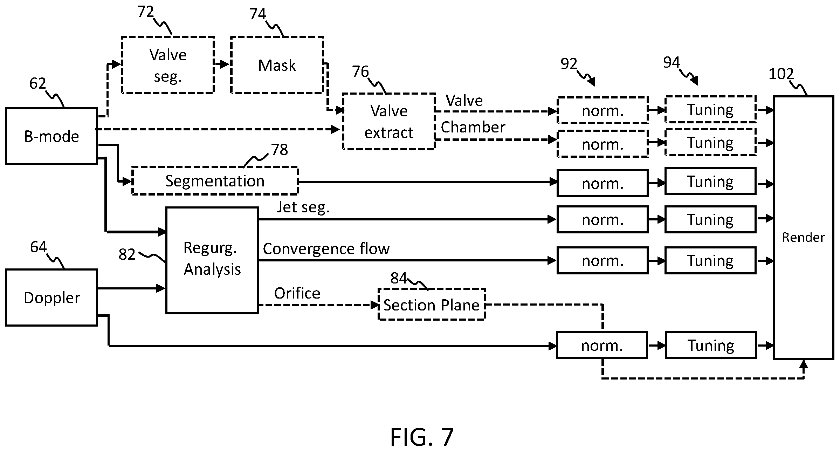

Fig. 7 outlines in block diagram form a process flow of an example method according to one particular set of embodiments.

The source 3D B-mode 62 and 3D Doppler 64 data are shown. At the final stage of the process, a renderer 102 processes input data to render an image that comprises a representation of each of the heart valve structure, the upstream converging flow region, and the output jet flow region.

Fig. 7 shows the previously discussed optional masking process for deriving the 3D raster representation of the valve, comprising segmenting the valve 72, applying voxel masking 74 to the segmented valve, extracting the valve signal from the masked data and thereby yielding a raster representation of the valve structure. Optionally, when generating the mask, the mask or the segmentation process may also identify or isolate regions corresponding to heart chambers (like the ventricles or atria). Similar steps may then be applied to extract signals corresponding to these areas. A single combined mask covering both the valve and one or more heart chamber regions may be generated, or separate masks for the valve and for the chamber region(s) may be generated.

This yields a raster representation of the valve, and optionally for the heart chamber region such as a ventricle and/or atrium.

Additionally or alternatively, segmentation 78 of the valve structure in the B-mode data 62 may be performed and rendering performed based on the segmentation without masking.

Also shown is the optional use of a regurgitation analysis module 82 adapted to output a geometry or other representation of the upstream convergence flow region and the output jet flow region, as discussed above. Optionally, the regurgitation analysis module 82 may also be configured to output a representation of a geometry of the valve orifice, based on the outer shape of the detected flow through the orifice.

In general terms, the regurgitation analysis module may be configured to receive at least Doppler ultrasound data, e.g. color Doppler data, and optionally also B-mode ultrasound data. The module may be configured to construct a 3D model of the orifice and/or the upstream converging flow region and/or the output jet flow region. The module may be configured to perform flow quantification, e.g. using techniques such as the Proximal Isovelocity Surface Area (PISA) method. This can be used to estimate the severity of regurgitation by calculating the flow rate across the valve.

An example flow analysis method which may be employed by the regurgitation analysis module 82 is described in detail in the document: WO 2023/020920 A1. The method disclosed therein receives as input both B-mode ultrasound data 62 and Doppler ultrasound data 64. The method comprises generating a model of the mitral valve based on the B-mode data. The method further comprises generating a model of the orifice based on the model of the mitral valve and the Doppler data. The method further comprises generating a blood flow model of the mitral regurgitation based on the model of the orifice. The method may further comprise comparing the blood flow model to the Doppler data and changing the model of the orifice such that the blood flow model is changed to match the Doppler data. For example, the model of the orifice may be changed to have at least one of a different shape, a different position, or a different orientation. The reader is referred to WO 2023/020920 A1 for a detailed description of implementation details for this example method for use by the regurgitation analysis module 82.

The output of the regurgitation analysis module may include at least a segmentation of the output jet flow, a geometry of the upstream converging flow region and optionally a geometry of the orifice of the valve.

In certain embodiments, the depiction of the heart valve structure 202, the upstream converging flow region 204, the output jet flow region 206 and/or the orifice can be tailored to show a sectional or cross-sectional view. This is achieved by defining a 2D sectional plane that cuts through these structures and rendering a view across this sectional plane. In the example of Fig. 7, the optional feature of rendering a sectional plane view of the orifice geometry output from the regurgitation analysis module is shown at block 84. However, rendering of a sectional view is also an optional feature for any of the heart valve structure 202, the upstream converging flow region 204, the output jet flow region 206 and/or the orifice geometry. Implementation details pertaining to this feature will be described in greater detail later.

Data normalization may be performed 92.

Rendering parameters for each element to be rendered may be independently adjusted or tuned 94, as discussed above.

A renderer 102 receives the raster and/or mesh representations of the heart valve structure, the output jet flow region, the upstream converging flow region and optionally the valve orifice, the left ventricle and/or the left atrium. The renderer is operable for rendering both 3D raster and mesh representations at the same time.

The valve and optionally the left atrium and the left ventricle are rendered by applying rendering to 3D raster representations obtained from the B-mode data.

With regards to the rendering, as discussed above, the upstream converging flow region and the output jet flow region can be rendered using a raster representation of the flow or using a mesh representation of the flow geometry. By way of example, for the isovelocity region, this may be rendered using a mesh representation. Alternatively, it may be rendered using a 3D raster representation formed from the voxels of the 3D color Doppler data. The color Doppler data may be first thresholded. Another alternative is to render using streamline representation.

By way of example, with regards to the output jet flow region, this may again be rendered using a mesh representation or using a 3D raster representation formed from the voxels of the 3D color Doppler data. The color Doppler data may be first thresholded. Another alternative is to represent the output jet flow region using a streamline representation.

In some embodiments, a combination of raster and mesh representations can be used in rendering the upstream converging flow region and/or the output jet flow region. This can be achieved for example by obtaining a segmentation of an outer geometry of the relevant flow region, and constructing a mesh of the outer geometry. The flow volume inside the outer geometry can be represented using a 3D raster formed form the 3D color Doppler voxels inside the region. The flow volume may then be rendered as a raster bounded by a mesh rendering of the outer geometry, i.e. a color Doppler representation of the fluid contained within a glass.

The renderer 102 generates a combined rendering of the different elements to be visualized, i.e. the mesh structure, the upstream converging flow region, the output jet flow region.

As discussed above, to optimize visibility of the visualization, various rendering parameters can be tuned. Tuning of the rendering parameters may be performed individually for each element to be included in the final rendered image. This is illustrated at block 94 of the flow diagram of Fig. 7.

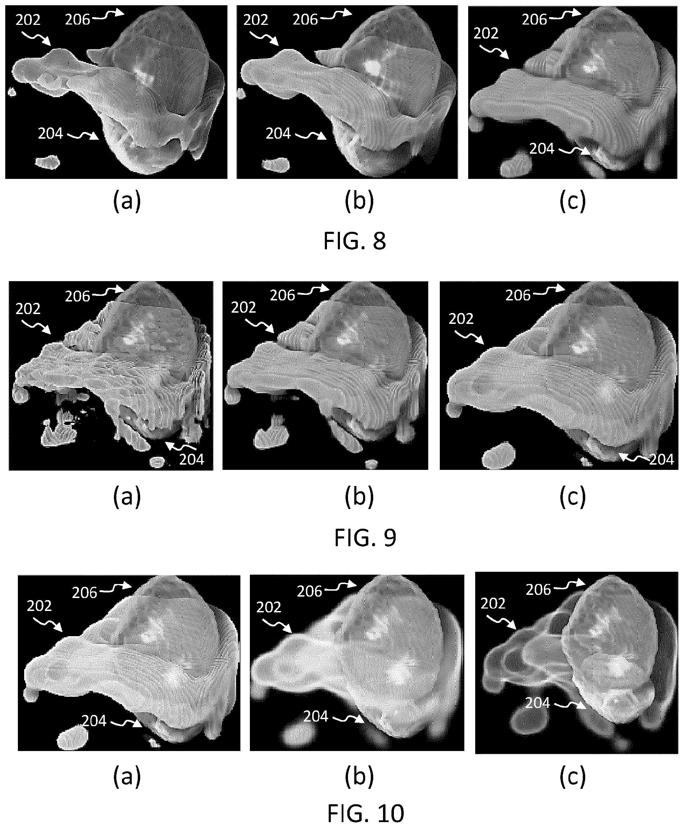

One possible parameter which may be adjusted is transparency. This can be adjusted for both mesh and raster renderings. Fig. 8 illustrates adjusting the transparency of the rendering of the heart valve structure 202. The heart valve structure 202, the upstream converging flow region 204 and output jet flow region 206 are all rendered. The transparency of the heart valve structure is adjusted from most transparent to least transparent from left to right (from Fig. 8(a)-(c)). It can be seen that where the valve structure 202 is rendered with greatest opacity, the upstream converging flow region 204 is least visible, but the geometry of the valve structure, particularly its surface morphology, is most clear. By contrast, at the left most image, the outer shape of the valve geometry is less clear but the shape of the upstream converging flow region 204 and the output jet flow region 206 is more visible. Thus, by adjusting the relative transparency of the renderings of each of the upstream converging flow region, the heart valve structure and the output jet flow region in the composite rendering, the visibility and appearance of the difference components can be configured. For example, a user, by adjusting the relative transparency, can select which elements to see more clearly and which to make more transparent.

Another possible parameter which may be adjusted is smoothness. This means adjusting the contours of the imaged elements to be more smooth and less noisy (at the cost of some resolution) or less smooth and more noisy. This parameter can be adjusted both for mesh and raster renderings. Fig. 9 illustrates adjusting the smoothness of the rendering of the heart valve structure 202. The heart valve structure 202, the upstream converging flow region 204 and output jet flow region 206 are all rendered. The smoothness of the heart valve structure 202 is adjusted from most least smooth to most smooth from left to right (from Fig. 9(a)-(c)).

Another possible parameter which may be adjusted is the fading. This means making some of the rendered elements more faded or less faded, i.e. more visible or less visible. For example, a relative fading of each of the heart valve structure, the upstream converging flow region and the output jet flow region (relative to one another) may be adjusted. By adjusting this parameter, this allows the user to adjust which elements are visible for making diagnostic assessments, e.g. fading out elements that are less important than others, and then fading them back in if needed. This parameter can be adjusted both for mesh and raster renderings. Fig. 10 illustrates adjusting the fading of the heart valve structure 202 in a rendering which includes the heart valve structure 202, the upstream converging flow region 204 and the output jet flow region 206. The fading of the heart valve structure 202 is adjusted from most least faded (most visible) to most faded from left to right (from Fig. 10(a)-(c)).

Another possible parameter which may be adjusted is the threshold setting(s) for 3D raster data rendering. By adjusting the threshold settings(s), this allows for dilating/shrinking the object represented by the 3D raster, e.g. the heart valve structure 202. In particular, in some embodiments, the threshold setting(s) may refer to cut-off values for voxels of the 3D raster above or below which voxels are excluded from the rendering.

In some embodiments, adjusting the threshold setting(s) may be implemented through adjusting a threshold setting of a transfer function used in rendering the relevant 3D raster. For example, in some embodiments, the rendering of a 3D raster representation may comprise applying a transfer function to voxels of the raster representation, wherein the transfer function defines one or more visualization properties, such as a transparency level, for each voxel of the raster as a function of voxel value. The one or more adjustable rendering parameters may include one or more parameters of the transfer function. The one or more adjustable rendering parameters may include a threshold setting of the transfer function.

For example, the rendering of the 3D raster may comprise application of a transfer function to the raster which defines the transparency level at which each voxel in the raster is to be rendered in the rendering. The transparency function may define the transparency for each voxel as a function of the voxel value (e.g. the intensity or density or Hounsfield value of each voxel in the case of B-mode ultrasound data). The possible transparency values may for example range between 0 (invisible) to 1 (opaque / non-transparent). In this context, the thresholding setting may comprise a threshold value which defines a voxel value above which the transfer function is greater than zero (i.e. is not invisible in the rendering). By setting a higher or lower threshold value, the rendered structure can appear as dilating/shrinking because more/fewer voxels are included in the rendering. The thresholding can be used to selectively include a certain type of imaged material in the rendered image and exclude another type of imaged material, for example to include voxels representing tissue regions and exclude voxels representing fluid regions.

In some examples, the thresholding setting could include an upper and lower threshold, where the lower threshold may determine the voxel value above which the transfer function is greater than zero (i.e. is not invisible in the rendering), and the upper threshold may define the voxel value above which the transfer function is 1 (i.e. is opaque / non-transparent).

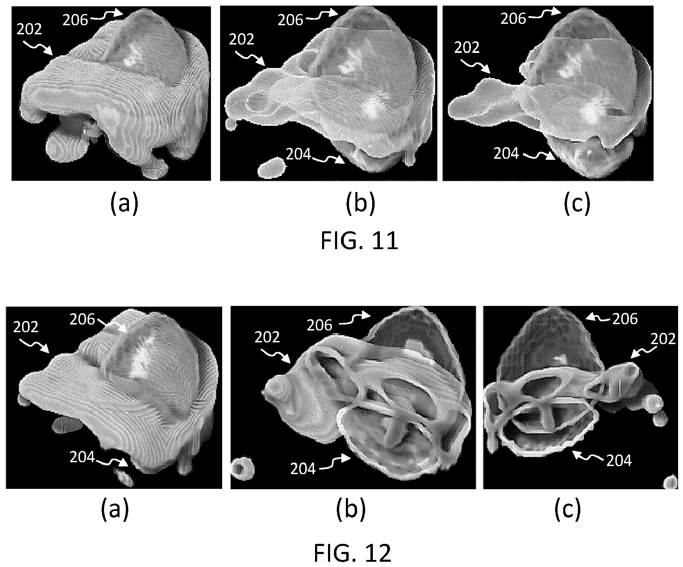

Fig. 11 illustrates adjusting the thresholding value used in rendering a raster representation of the heart valve structure 202 in a rendering which includes the heart valve structure 202, the upstream converging flow region 204 and the output jet flow region 206. The thresholding value of the heart valve structure 202 is adjusted from highest to lowest from left to right (from Fig. 11 (a)-(c)).

In some embodiments, the adjustable rendering parameters may include a color tinting of each of the heart valve structure 202, the upstream converging flow region 204 and the output jet flow region 206. This makes the different elements easier to distinguish in the visualization. Thus, the different elements are parametrized by colors. In some embodiments, the color tinting may be applied just to the mesh renderings. This would allow to better differentiate them from the raster renderings.

In some embodiments, the composite rendering of the heart valve structure 202, the upstream converging flow region 204 and output jet flow region 206 may be further configured to represent a sectional view through the rendered elements, i.e. where a 2D cut plane is defined through the structures and a visualization is rendered which represents a volume rendering of a virtual cut-away view as though viewed through the cut plane.

As mentioned above, in certain embodiments, the depiction of the heart valve structure 202, the upstream converging flow region 204, and the output jet flow region 206 can be tailored to show a sectional or cross-sectional view. This is achieved by defining a 2D plane that cuts through these structures. The resulting visualization then represents a volume-rendered image, giving an observer the impression of seeing inside these elements, as if looking through the specified cut plane. This allows for showing what is inside and avoiding superposing elements that mask one another. The section plane position may be adjusted so as to optimize a view of the mitral regurgitation from the point of view of a particular diagnostic application.

This sectional view rendering, often called cross-sectional or cutaway rendering, is a visualization technique that exposes the inner details of a structure that might otherwise be hidden from view. This is accomplished by virtually 'cutting' away or 'slicing' through parts of the structure, allowing for a clear view of its internal components. The process comprises defining a 2D cut plane through which the structure will be sectioned. Once the plane is defined, any material or structure on one side of this plane is virtually removed or made transparent in the rendering process. A volume-rendered image of the remaining portion of the structure can then be generated.

The position and angle of the cut plane can be defined in different ways. One option is to permit adjustment by a user of the position and the orientation of the plane. A further option is to define the orientation and/or position of the cut plane relative to the long axis of the valve (or the orifice) and its normal axis. A further option is to define the orientation and/or position of the cut plane relative to the long axis of the valve (or the orifice) and the direction of the jet. A further option is to define the orientation and/or position of the cut plane relative to the orientation axis of all objects (valve, orifice, jet, convergence flow).

In some embodiments, the cut plane could be configured so as to move with movement of the anatomy. For example, if the hole in the valve is moving and changing its axis, it is possible to define a dynamic cut plane which follows the center of the hole during the acquisition. Its orientation could be constrained to a compromise of visibility of the valve, visibility of incoming flow, visibility output jet, and low oscillation from one frame to another.

In a case where several holes are present in the valve, the cut plane position and orientation may be selected to represent a compromise between cutting through all the holes and not moving too much. In case of two holes, this will be the plane going through both. Usually for mitral regurgitation more than two holes is rare.

The camera point of view may be for example directed front of the sectional plane and aligned to the center of the valve.

Fig. 12 illustrates rendering of sectional views of the heart valve structure 202, upstream converging flow region 204, and output jet flow region 206. Fig. 12(a) shows a non-sectional volume rendering of the three elements. Fig. 12(b) shows a sectional view across a cut plane which extends through a middle of the heart valve structure. Fig. 12(b) shows a sectional view across the same cut plane from the opposite side.

Although examples discussed above have referred to application of the visualization method for assessing mitral regurgitation, the same method can be applied for assessing any other type of flow defect.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method (10) for cardiac valve visualization using ultrasound data, comprising:
receiving (12) 3D B-mode ultrasound data of a cardiac region containing a heart valve structure;
receiving (14) 3D Doppler ultrasound data of the cardiac region;
generating a 2D image, wherein generating the 2D image comprises:
identifying (16) a 3D region of the B-mode data occupied by the heart valve structure to yield a 3D raster representation of the heart valve;
identifying (18) an output jet flow region within the cardiac region containing a fluid jet output from the heart valve, based on processing of at least the Doppler ultrasound data;
identifying (20) an upstream converging flow region within the cardiac region, wherein an upstream converging flow region is a region containing at least an isovelocity surface of blood flow, based on processing of at least the Doppler ultrasound data;
performing (26) a rendering process to render a 2D image which includes a visualization of each of the heart valve structure, the output jet flow region, and the upstream converging flow region,
wherein the rendering process includes applying rendering to the 3D raster representation of the heart valve for realizing in the 2D image a 2D visualization of the heart valve structure.

2. The method of claim 1, wherein the method comprises independently adjusting (22) one or more rendering parameters applied in rendering each of the heart valve structure, the output jet flow region, and the upstream converging flow region.

3. The method of claim 2, wherein the one or more parameters include at least a transparency level of each of the heart valve structure, the output jet flow region, and the upstream converging flow region in the generated 2D image.

4. The method of any of claims 2-3, wherein the one or more rendering parameters include one or more of: transparency, smoothness, color, and/or fading applied to each of the heart valve structure, the output jet flow region, and the upstream converging flow region.

5. The method of any of claims 2-4, wherein the adjusting the one or more rendering parameters comprises receiving user input from a user interface indicative of settings for at least one of the one or more rendering parameters and adjusting the at least one rendering parameter based on the user input.

6. The method of any of claims 1-5, wherein the rendering the heart valve structure comprises applying a transfer function to voxels of the raster representation of the heart valve, wherein the transfer function defines a transparency level for each voxel of the raster as a function of voxel value, and wherein the one or more rendering parameters include one or more parameters of the transfer function.

7. The method of any of claims 1-6,
wherein the identifying the output jet flow region comprises segmenting the output jet flow region in the Doppler ultrasound data; and
wherein the rendering of the output jet flow region comprises:
generating a 3D mesh of an outline of the output jet flow region based on the segmentation, and
applying volume rendering to the mesh.

8. The method of any of claims 1-6, wherein the rendering of the output jet flow region comprises applying volume rendering to a 3D raster representation of the output jet flow region within the Doppler data.

9. The method of any of claims 1-8,
wherein the identifying the upstream converging flow region comprises segmenting the upstream converging flow region in the Doppler data;
wherein the rendering of the upstream converging flow region comprises:
generating a 3D mesh of the upstream converging flow region based on the segmentation;
applying volume rendering to the mesh.

10. The method of any of claims 1-8, wherein the rendering the upstream converging flow region comprises applying volume rendering to a 3D raster representation of the upstream converging flow region within the Doppler data.

11. The method of any preceding claim, wherein the identifying the 3D region of the B-mode data occupied by the heart valve structure comprises:
segmenting the heart valve structure in the B-mode data, and
computing a 3D mask based on the segmentation, the 3D mask distinguishing the 3D region occupied by the heart valve structure from a background region.

12. The method of any preceding claim, wherein the identifying of the output jet flow region and the upstream converging flow region comprises applying a flow dynamics model to the Doppler ultrasound data, the flow dynamics model adapted to:
determine an isovelocity surface of blood flow upstream of the heart valve;
determine a blood flow path through the valve;
determine an output jet from the valve.

13. A processing device (32), comprising one or more processors (36) configured to:
receive: 3D B-mode ultrasound data of a cardiac region containing a heart valve structure, and receive 3D Doppler ultrasound data of the cardiac region;
identify a 3D region of the B-mode data occupied by the heart valve structure to yield a 3D raster representation of the heart valve;
identify an output jet flow region within the cardiac region containing a fluid jet output from the heart valve, based on processing of at least the Doppler ultrasound data;
identify an upstream converging flow region within the cardiac region, wherein an upstream converging flow region is a region containing at least an isovelocity surface of blood flow, based on processing of at least the Doppler ultrasound data; and
perform a rendering process to render a 2D image which includes a visualization of each of the heart valve structure, the output jet flow region, and the upstream converging flow region,
wherein the rendering process includes applying rendering to the 3D raster representation of the heart valve for realizing in the 2D image a 2D visualization of the heart valve structure.

14. The device of claim 13, wherein the one or more processors are further configured to independently adjust one or more rendering parameters applied in rendering each of the heart valve structure, the output jet flow region, and the upstream converging flow region.

15. A system (30), comprising:
the processing device (32) of claim 13 or 14; and
an ultrasound acquisition apparatus (54), operable to acquire 3D B-mode data and 3D Doppler data.
